# EUROPÄISCHE PATENTSCHRIFT

(11) **EP 1 889 579 B1**
(45) Veröffentlichungstag und Bekanntmachung des Hinweises auf die Patenterteilung: **28.12.2011**
(21) Anmeldenummer: 07015338.2
(22) Anmeldetag: 06.08.2007
(51) Int. Cl.: A61B 17/28

(54) **Medizinisches Rohrschaftinstrument**
Medical tube shaft instrument
Instrument médical à tige cylindrique

(30) Priorität: 17.08.2006 DE 102006038516
(43) Veröffentlichungstag der Anmeldung: 20.02.2008
(73) Patentinhaber: Karl Storz GmbH & Co. KG, 78532 Tuttlingen (DE)
(72) Erfinder: Renger, Uwe, 78247 Hilzingen (DE); Blocher, Martin, 78532 Tuttlingen (DE); Bacher, Uwe, 78532 Tuttlingen (DE)
(74) Vertreter: Hofmeister, Frank

(56) Entgegenhaltungen:
- DE-U1- 9 317 535
- DE-U1-202007 003 114
- US-A- 5 478 347
- US-A- 5 603 723
- US-A- 5 893 875

## Beschreibung

Die Erfindung betrifft ein medizinisches Rohrschaftinstrument mit einem hohlen Schaft, einer am proximalem Ende des Schaftes angeordneten Handhabe und mindestens einem in dem hohlen Schaft gelagerten Werkzeugbetätigungselement, an dessen distalem Ende ein aus zwei Maulteilen bestehendes Werkzeug angeordnet ist, wobei das Werkzeugbetätigungselement zum Betätigen des Werkzeugs mit mindestens einem Betätigungsmechanismus der Handhabe koppelbar ist und wobei das Werkzeugbetätigungselement und der hohle Schaft über einen Kopplungsmechanismus lösbar miteinander verbindbar sind.

Derartige medizinische Rohrschaftinstrumente finden beispielsweise in der Ausbildung als Nadelhalter Verwendung in der endoskopischen Chirurgie. Aufgrund steigender hygienischer Anforderungen wird immer häufiger gefordert, dass insbesondere Hohlräume, wie beispielsweise hohle Schäfte; aufweisende Rohrschaftinstrumente zumindest teilweise zerlegbar ausgebildet sind, um diese einer gründlichen Reinigung und Sterilisation, vorzugsweise Dampfsterilisation, unterziehen zu können.

Ein gattungsgemäßes medizinisches Rohrschaftinstrument ist beispielsweise aus der US 5 603 723 A bekannt das die Merkmale des Oberbegriffs von Anspruch 1 offenbart.

Aus der DE 43 07 539 A1 ist ein weiteres, als medizinische Zange ausgebildetes medizinisches Rohrschaftinstrument bekannt. Dieses bekannte Rohrschaftinstrument lässt sich zum Reinigen und Sterilisieren in drei Hauptgruppen zerlegen, das als Zug-/Schubstange ausgebildete Werkzeugbetätigungselement, den hohlen Schaft sowie die Handhabe. Der Kopplungsmechanismus zum Verbinden von Zug-/Schubstange und hohlem Schaft ist bei dieser Konstruktion als Bajonettverbindung ausgebildet.

Zwar haben sich diese Bajonettverbindungen in der Praxis bewährt, jedoch bedarf die Handhabung bei der Montage des Instruments einer gewissen Übung.

Davon ausgehend liegt der Erfindung die Aufgabe zugrunde, ein medizinisches Rohrschaftinstrument derart weiterzubilden, dass das Werkzeugbetätigungselement und der hohle Schaft über einen im Wesentlichen spielfreien und einfach zu handhabenden Kopplungsmechanismus lösbar miteinander verbindbar sind.

Die Lösung dieser Aufgabenstellung ist erfindungsgemäß dadurch gekennzeichnet, dass die Arretierung des Kopplungsmechanismus zwischen dem Werkzeugbetätigungselement und dem hohlen Schaft in Abhängigkeit von der Stellung der Maulteile des Werkzeugs zueinander erfolgt, wobei das Werkzeugbetätigungselement ausschließlich in einer von den Arbeitspositionen abweichenden Montagestellung des Werkzeugs über den Kopplungsmechanismus mit dem hohlen Schaft verbindbar ist.

Durch diese erfindungsgemäße Ausgestaltung des Kopplungsmechanismus, der vorteilhafterweise so ausgebildet ist, dass das Werkzeugbetätigungselement ausschließlich in einer von den Arbeitspositionen abweichenden Montagestellung des Werkzeugs über den Kopplungsmechanismus mit dem hohlen Schaft verbindbar ist, wird sichergestellt, dass ein versehentliches Lösen der Verbindung zwischen Werkzeugbetätigungselement und hohlem Schaft während des normalen Arbeitsbetriebs ausgeschlossen ist, da das Werkzeug diese Montagestellung nur in einem zumindest teildemontierten Zustand des Instruments einnehmen kann.

Gemäß einer ersten praktischen Ausführungsform der Erfindung ist der Kopplungsmechanismus in der Art einer Bajonettverbindung ausgebildet.

Mit einer zweiten erfindungsgemäßen Ausgestaltung wird vorgeschlagen, dass der Kopplungsmechanismus als selbstsichernde Zapfen-Schlitz-Steuerung ausgebildet ist.

Durch die selbstsichernde Ausgestaltung der Zapfen-Schlitz-Steuerung wird sichergestellt, dass nach erfolgter Kopplung der beiden miteinander zu verbindenden Bauteile diese sich beispielsweise bei der weiteren Montage des Instruments nicht versehentlich wieder voneinander lösen können. Hierdurch wird die Montage deutlich vereinfacht und auch für ungeübte Benutzer einfach und sicher durchführbar.

Zur Ausbildung der selbstsichernden Kopplung wird mit der Erfindung vorgeschlagen, dass die Zapfen-Schlitz-Steuerung aus mindestens einem an einem der miteinander zu koppelnden Bauteile ausgebildeten Steuerzapfen und mindestens einer am jeweils anderen Bauteil ausgebildeten Führungsbahn zur Aufnahme eines Steuerzapfens besteht und dass mindestens ein in eine Führungsbahn eingesetzter Steuerzapfen in der Arbeitsposition des Werkzeugs gegen Entnahme aus der Führungsbahn blockiert ist.

Gemäß einer praktischen Ausführungsform der Erfindung ist der mindestens eine Steuerzapfen distalseitig an der Innenseite des hohlen Schaftes ausgebildet. Vorzugsweise ist an der Innenseite des distalseitigen Ende des hohlen Schaftes ein Steuerzapfen ausgebildet und sind am distalseitigen Ende des Werkzeugbetätigungselements zwei einander gegenüberliegende Führungsbahnen zur Aufnahme des Steuerzapfens ausgebildet. Die Verwendung von zwei einander gegenüberliegende Führungsbahnen auch bei nur einem Steuerzapfen hat den Vorteil, dass die beiden miteinander zu koppelnden Bauteile nicht nur in einer einzigen Stellung der Bauteile zueinander miteinander koppelbar sind, wodurch die Montage vereinfacht wird. Selbstverständlich ist es auch möglich, zwei Steuerzapfen und zwei Führungsbahnen vorzusehen, wobei die Steuerzapfen und Führungsbahnen einander entsprechend am Umfang des Schaftes bzw. des Werkzeugbetätigungselements angeordnet sein müssen.

Der Betätigungsmechanismus der Handhabe, über den das Werkzeugbetätigungselement und somit auch das Werkzeug betätigbar sind, ist erfindungsgemäß vorteilhafterweise als verschwenkbar an der Handhabe gelagerter Griffteil ausgebildet.

Mit einer weiteren praktischen Ausgestaltung der Erfindung wird vorgeschlagen, dass das Werkzeug als aus mindestens zwei Maulteilen bestehendes Werkzeug ausgebildet ist, wobei mindestens ein Maulteil des Werkzeugs über das Werkzeugbetätigungselement gegenüber dem anderen Maulteil verschwenkbar ist.

Gemäß einer bevorzugten Ausführungsform der Erfindung wird vorgeschlagen, dass das Werkzeugbetätigungselement als Zug- Druckstange ausgebildet ist.

Weiterhin wird mit der Erfindung vorgeschlagen, dass dieses als Zug-/Schubstange ausgebildete Werkzeugbetätigungselement aus einem Stangengrundkörper und einem mit dem Stangengrundkörper fest verbindbaren, das distale Ende der Zug-/Schubstange bildenden Werkzeugeinsatz besteht. Die zweiteilige Ausgestaltung der Zug-/Schubstange ermöglicht es, dass für die Ausgestaltung verschiedenster Rohrschaftinstrumente ein immer gleichbleibender und nur in der Länge unterschiedlicher Stangengrundkörper hergestellt werden muss. Zur Endfertigung eines konkreten Rohrschaftinstruments ist es dann nur noch notwendig, den jeweils erforderlichen Werkzeugeinsatz mit dem Stangengrundkörper, beispielsweise durch Verschweißen, fest zu verbinden.

Gemäß einer bevorzugten Ausführungsform der Erfindung wird vorgeschlagen, dass der Werkzeugeinsatz aus einer mit einem starren Maulteil des Werkzeugs versehenen Hülse und einem verschiebbar in der Hülse gelagerten Werkzeugschaft besteht, der einerseits mit einem verschwenkbaren Maulteil des Werkzeugs gekoppelt ist und andererseits fest mit dem Stangengrundkörper verbindbar ist, wobei die Führungsbahn zur Aufnahme eines Steuerzapfens vorzugsweise in der Hülse des Werkzeugeinsatzes ausgebildet ist. Diese konstruktive Ausgestaltung, bei der sich die einstückig mit dem starren Maulteil ausgebildete Hülse am hohlen Schaft abstützt und der verschiebbar in der Hülse gelagerte Werkzeugschaft fest mit dem Stangengrundkörper der Zug-/Schubstange verbunden ist, ermöglicht eine direkte und spielfreie Kraftübertragung der über die Handhabe auf die Zug-/Schubstange aufgebrachten Zug- oder Schubkräfte auf das verschwenkbare Maulteil des Werkzeugs.

Vorzugsweise ist die Führungsbahn wendelförmig ausgebildet und weist erfindungsgemäß einen Steigungswinkel α auf, der in Abhängigkeit von der Reibungspaarung eine vorbestimmte maximale Steigung von vorzugsweise 45° nicht übersteigen soll, um eine Selbsthemmung des Steuerzapfens in der Führungsbahn während der Montage bzw. Demontage zu verhindern.

Um sicherzustellen, dass das Werkzeugbetätigungselement und der hohle Schaft ausschließlich in einer von den Arbeitsstellungen abweichenden Montagestellung des Werkzeugs miteinander koppelbar und wieder lösbar sind, wird mit der Erfindung vorgeschlagen, dass im Bereich des in der Handhabe gelagerten proximalen Endes des Werkzeugbetätigungselements und des hohlen Schaftes eine die Verlagerbarkeit des Werkzeugbetätigungselements innerhalb des hohlen Schaftes limitierende Begrenzungsvorrichtung angeordnet ist. Diese Begrenzungsvorrichtung limitiert einerseits die Wegstrecke der Axialverschiebung des Werkzeugbetätigungselements innerhalb des hohlen Schaftes, so dass dieses nur soweit verschiebbar ist, dass das Werkzeug von der geschlossenen Arbeitsstellung in die geöffnete Arbeitsstellung und zurück verstellbar ist, und andererseits verhindert die Begrenzungsvorrichtung eine Rotation des Werkzeugbetätigungselements um seine Längsachse, wenn dieses nicht als Torsionselement ausgebildet ist.

Die erfindungsgemäße Begrenzungsvorrichtung besteht vorzugsweise aus mindestens einer am Werkzeugbetätigungselement ausgebildeten Anlagefläche und einem mit dieser Anlagefläche in Wirkverbindung bringbaren, im hohlen Schaft gelagerten Anlageelement.

Mit einer praktischen Ausführungsform zur Ausgestaltung der Begrenzungsvorrichtung wird vorgeschlagen, dass am Werkzeugbetätigungselement zwei einander gegenüberliegende, als Ausnehmungen ausgebildete Anlageflächen angeordnet sind und in einer Ausnehmung im hohlen Schaft ein Anlageelement gelagert ist, das im montierten Zustand von Werkzeugbetätiglungselement und hohlem Schaft im Wesentlichen formschlüssig an einer der Anlageflächen des Werkzeugbetätigungselements anliegt. Das im Wesentlichen formschlüssig mit einer der Anlageflächen des Werkzeugbetätigungselements zusammenwirkende Anlageelement blockiert im mit der Handhabe verbundenen Montagezustand von Werkzeugbetätigungselement und hohlem Schaft eine Axialverschiebung des Werkzeugbetätigungselements über das Maß hinaus, das zur Überführung des Werkzeugs in die geöffnete Arbeitsstellung erforderlich ist. Ein versehentliches Überführen des Werkzeugs in die Montagestellung wird durch die erfindungsgemäße Begrenzungsvorrichtung zuverlässig verhindert.

Weiterhin wird mit der Erfindung vorgeschlagen, dass der Werkzeugschaft und das verschwenkbare Maulteil über mindestens einen beidseitig verschwenkbar gelagerten Gelenkhebel miteinander verbunden sind, um eine bessere Kraftübertragung auf das Maulteil zu gewährleisten.

Um den vollständig in die Führungsbahn eingesetzten Steuerzapfen im miteinander gekoppelten Zustand von hohlem Schaft und Werkzeugbetätigungselement zu sichern, wird erfindungsgemäß vorgeschlagen, dass im Werkzeugschaft des Werkzeugeinsatzes eine in Axialrichtung des Werkzeugschaftes verlaufende proximalseitig offene Ausnehmung zur Aufnahme des Steuerzapfens ausgebildet ist, wobei mindestens eine der in Axialrichtung des Werkzeugschaftes im Wesentlichen parallel zueinander verlaufenden Kanten der Ausnehmung in der Arbeitsstellung des Werkzeugs eine begrenzende Anschlagfläche für den Steuerzapfen bildet.

Schließlich wird mit der Erfindung vorgeschlagen, dass das Werkzeug zum Entkoppeln des Werkzeugbetätigungselements vom hohlen Schaft über einen leichten "Schlag" auf das von der Handhabe entkoppelte proximale Ende des Werkzeugbetätigungselements in die Montagestellung überführbar ist. Ebenso ist es möglich, das Werkzeug per Hand, beispielsweise durch Drücken auf das von der Handhabe entkoppelte proximale Ende des Werkzeugbetätigungselements relativ zum hohlen Schaft in die Montagestellung zu überführen. Diese Art der Überführung des Werkzeugbetätigungselements in die Montagestellung stellt sicher, dass das erfindungsgemäße Rohrschaftinstrument niemals im zusammengesetzten Gebrauchszustand versehentlich in die den hohlen Schaft und das Werkzeugbetätigungselement trennende Montagestellung überführbar ist.

Mit einer weiteren Ausbildungsform wird erfindungsgemäß vorgeschlagen, dass das als Zug- Druckstange ausgebildete Werkzeugbetätigungselement aus einem Stangengrundkörper und einem mit dem Stangengrundkörper fest verbindbaren, das distale Ende der Zug-/Schubstange bildenden Werkzeugeinsatz besteht, wobei der Werkzeugeinsatz aus einer mit einem starren Maulteil des Werkzeugs versehenen Hülse und einem in der Hülse angeordneten, das distale Ende der Zug-/Schubstange koaxial umgebenden Führungseinsatz besteht.

Um im montierten Zustand ein Verdrehen der Zug-/Schubstange und somit auch der Maulteile zu verhindern, was zum Lösen der Bajonettverbindung führen würde, weist die Zug-/Schubstange erfindungsgemäß im Bereich ihres in dem Führungseinsatz gelagerten distalen Endes einen quer zur Längsachse der Zug-/Schubstange angeordneten Verriegelungsstift aufweist, der frei beweglich so in einer Bohrung der Zug-/Schubstange gelagert ist, dass die freien Enden des Verriegelungsstiftes im Führungseinsatz ausgebildete Führungsbahnen durchragen und somit eine Drehbewegung blockieren.

Weiterhin wird mit der Erfindung vorgeschlagen, dass die freien Enden des Verriegelungsstiftes als bolzenkopfförmige Verdickungen ausgebildet sind, um ein Herausrutschen des Verriegelungsstiftes aus den Führungsbahnen und der Bohrung verhindern.

Weitere Merkmale und Vorteile der Erfindung ergeben sich anhand der zugehörigen Zeichnung, in der zwei Ausführungsbeispiele eines erfindungsgemäßen medizinischen Rohrschaftinstruments nur beispielhaft dargestellt sind, ohne die Erfindung auf diese Ausführungsbeispiele zu beschränken. In der Zeichnung zeigt:
- Fig. 1: eine schematische Seitenansicht eines erfindungsgemäßen Rohrschaftinstruments;
- Fig. 2: eine schematische Seitenansicht des Instruments gemäß Fig. 1 im zerlegten Zustand;
- Fig. 3: eine teilweise geschnittene schematische Seitenansicht der Zug/Schubstange gemäß Fig. 2, jedoch das Werkzeug in der geschlossenen Arbeitsstellung darstellend;
- Fig. 4: eine vergrößerte und teilweise geschnittene Seitenansicht einer ersten Ausführungsform des Details IV gemäß Fig. 1, jedoch das Werkzeug in der Montagestellung darstellend;
- Fig. 5: eine vergrößerte distalseitige Vorderansicht des hohlen Schaftes in Richtung V-V gemäß Fig. 2;
- Fig. 6a: eine vergrößerte und teilweise geschnittene Seitenansicht der ersten Ausführungsform des Details IV gemäß Fig. 1, jedoch das Werkzeug in der Montagestellung darstellend;
- Fig. 6b: eine Darstellung gemäß Fig. 6a, das Werkzeug in der geöffneten Arbeitsstellung darstellend;
- Fig. 6c: eine Darstellung gemäß Fig. 6a, jedoch das Werkzeug in der geschlossenen Arbeitsstellung darstellend;
- Fig. 7: eine teilweise geschnittene schematische Draufsicht des Details VII gemäß Fig. 2;
- Fig. 8a: eine vergrößerte und geschnittene Seitenansicht einer zweiten Ausführungsform des Details VIII gemäß Fig. 1, jedoch das Werkzeug in der Montagestellung darstellend;
- Fig. 8b: eine ungeschnittene Draufsicht auf die Darstellung gemäß Fig. 8a;
- Fig. 9a: eine vergrößerte und geschnittene Seitenansicht der zweiten Ausführungsform des Details VIII gemäß Fig. 1, das Werkzeug in der geöffneten Arbeitsstellung darstellend;
- Fig. 9b: eine ungeschnittene Draufsicht auf die Darstellung gemäß Fig. 9a und
- Fig. 9c: eine Darstellung gemäß Fig. 9a, jedoch das Werkzeug in der geschlossenen Arbeitsstellung darstellend.

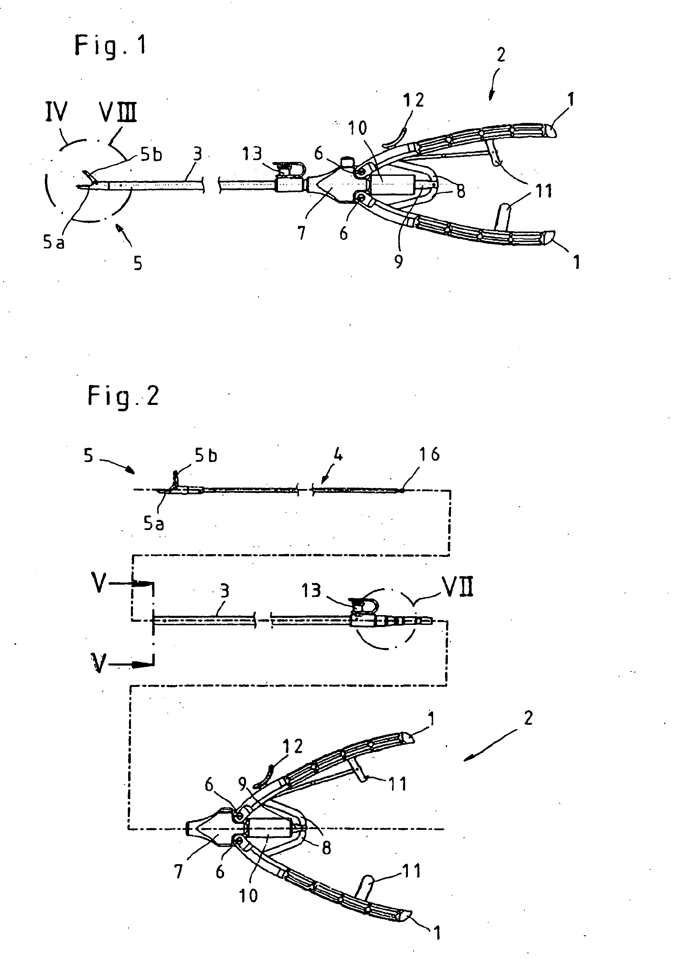

Das in den Abbildungen Fig. 1 und 2 dargestellte, als Nadelhalter ausgebildete medizinische Rohrschaftinstrument besteht im Wesentlichen aus einer mit zwei Griffteilen 1 versehenen Handhabe 2, einem hohlen Schaft 3 sowie einem in den hohlen Schaft 3 einsetzbaren Werkzeugbetätigungselement 4, an dessen distalem Ende ein aus zwei Maulteilen 5a und 5b bestehendes Werkzeug 5 angeordnet ist. Bei dem dargestellten Ausführungsbeispiel ist das Werkzeugbetätigungselement 4 als Zug-/Schubstange 4 ausgebildet. Selbstverständlich sind auch andere Ausgestaltungen des Werkzeugbetätigungselements 4 verwendbar, wie beispielsweise die Ausgestaltung als Torsionsstab.

Die in Fig. 2 besonders deutlich dargestellten Baugruppen Handhabe 2, hohler Schaft 3 und Zug-/Schubstange 4 sind über Kopplungs- und Rastmechanismen so miteinander koppelbar, dass durch Betätigen der Griffteile 1 der Handhabe 2 die Maulteile 5a und 5b des Werkzeugs 5 zwischen einer offenen und einer geschlossenen Arbeitsstellung verstellbar sind, wobei die vom Benutzer auf die Griffteile 1 der Handhabe 2 aufgebrachten Kräfte über die Zug-/Schubstange 4 auf die Maulteile 5a, 5b des Werkzeugs 5 übertragen werden.

Alternativ zu der dargestellten Ausgestaltung der Handhabe 2 mit zwei Griffteilen 1 ist es auch möglich, die Zug-/Schubstange 4 beispielsweise über einen an der Handhabe 2 gelagerten Betätigungsmechanismus in Form eines axial verlagerbaren Schiebers zu betätigen. Ebenso kann das Werkzeug 5 beispielsweise aus einem mit der Zug-/Schubstange 4 koppelbaren Messer bestehen, das über die Zug-/Schubstange 4 ausschließlich axial verschiebbar ist.

Wie aus Fig. 1 und 2 ersichtlich, sind bei der dargestellten Ausführungsform beide Griffteile 1 der Handhabe 2 als verschwenkbare Griffteile 1 ausgebildet, die über Anlenkpunkte 6 verschwenkbar an einem Gehäuse 7 der Handhabe 2 gelagert sind. Zur Überführung der Schwenkbewegung der Griffteile 1 in eine reine Axialbewegung der Zug-/Schubstange 4 sowie zur Kraftübertragung der vom Benutzer über die Handhabe 2 eingeleiteten Zug- und/oder Druckkraft auf die Zug-/Schubstange 4 sind beide Griffteile 1 über jeweils einen Gelenkhebel 8 mit einer Kupplungsstange 9 verbunden, die ihrerseits direkt oder indirekt über einen Kopplungsmechanismus mit der Zug-/Schubstange 4 gekoppelt ist, wobei die Kopplung der Zug-/Schubstange 4 mit der Kupplungsstange 9 und somit mit der Handhabe 2 in dem Kupplungsgehäuse 10 erfolgt.

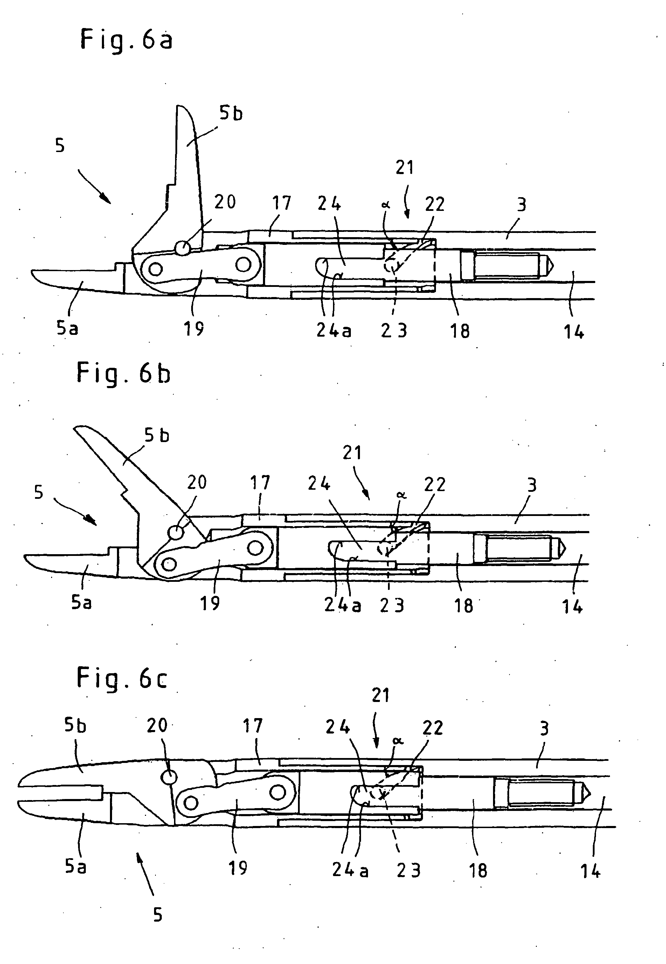

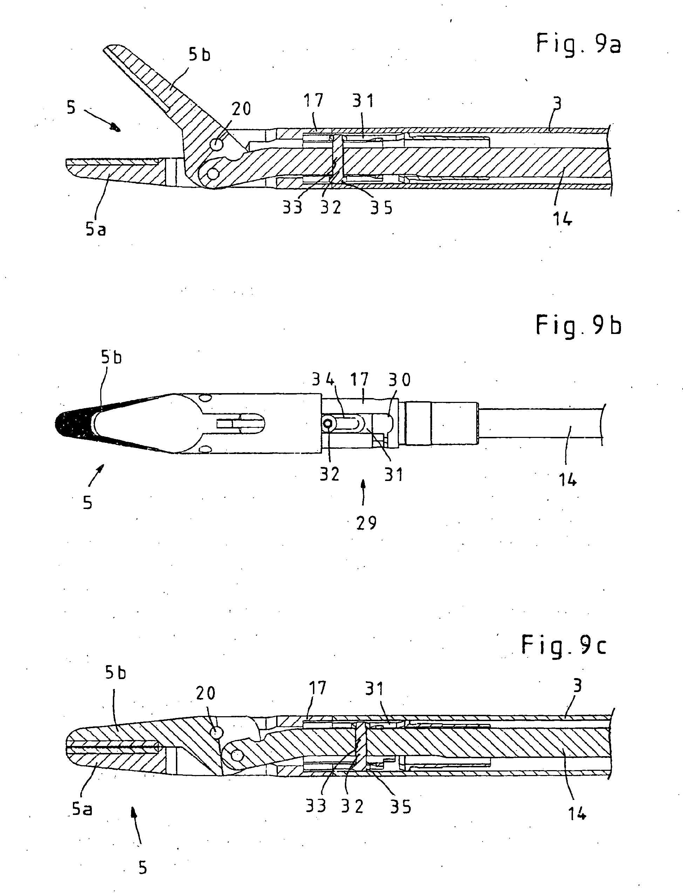

Die Kopplung der Zug-/Schubstange 4 und somit auch der Maulteile 5a und 5b des Werkzeugs 5 mit den Griffteilen 1 der Handhabe 2 ist so ausgelegt, dass beim Zusammendrücken der Griffteile 1 die Zug-/Schubstange 4 über die Gelenkhebel 8 und die Kupplungsstange 9 in axialer Richtung zum proximalen Ende des Instruments gezogen wird. Diese Axialverschiebung der Zug-/Schubstange 4 zum proximalen Ende des Instruments bewirkt ein Überführen der Maulteile 5a, 5b des Werkzeugs 5 in die geschlossene Arbeitsstellung, wie diese in Fig. 6c und 9c dargestellt ist. In dieser zusammengedrückten Stellung sind die Griffteile 1 über eine Arretiervorrichtung 11 gegeneinander fixierbar, so dass der Benutzer nicht die gesamte Zeit die Druckkraft auf die Griffteile 1 der Handhabe 2 ausüben muss. Über einen Entriegelungsknopf 12, der die Teile der Arretiervorrichtung 11 trennt, ist diese Fixierung wieder aufhebbar.

Alternativ zu der dargestellten Ausführungsform ist es aber auch möglich, die Kopplung der Zug-/Schubstange 4 und somit auch der Maulteile 5a und 5b des Werkzeugs 5 mit den Griffteilen 1 der Handhabe 2 so auszugestalten, dass eine Axialverschiebung der Zug-/Schubstange 4 zum distalen Ende des Instruments ein Überführen der Maulteile 5a, 5b des Werkzeugs 5 in die geschlossene Arbeitsstellung bewirkt.

Vorteilhafterweise sind die Griffteile über ein Federelement in die Offenstellung vorgespannt, das beispielsweise im Kupplungsgehäuse 10 angeordnet sein kann. Sobald der Entriegelungsknopf 12 betätigt wird, schiebt dann dieses Federelement die Zug-/Schubstange 4 in axialer Richtung zum distalen Ende des Instruments, wodurch die Griffteile 1 über die Kupplungsstange 9 und die Gelenkhebel 8 auseinander gedrückt werden. Diese Axialverschiebung der Zug-/Schubstange 4 zum distalen Ende des Instruments bewirkt ein Überführen der Maulteile 5a, 5b des Werkzeugs 5 in die offene Arbeitsstellung, wie diese in Fig. 6b und 9a dargestellt ist.

Alternativ zu der dargestellten Ausführungsform der Handhabe 2 mit zwei verschwenkbaren Griffteilen 1 ist es selbstverständlich auch möglich, nur ein Griffteil 1 verschwenkbar auszubilden, wohingegen das andere Griffteil dann beispielsweise einstückig starr mit dem Gehäuse 7 der Handhabe 2 ausgebildet ist. Bei einer solchen Ausgestaltung ist es möglich, die Zug-/Schubstange 4 direkt mit dem verschwenkbaren Griffteil 1 zu koppeln.

Weiterhin ist es möglich, im Kopplungsbereich der Zug-/Schubstange 4 mit der Handhabe 2 eine Überlastsicherung vorzusehen, die eine zu große Krafteinleitung in die Zug-/Schubstange 4 verhindert. Eine solche Überlastsicherung kann beispielsweise als Überlastfeder ausgebildet im Bereich der Kupplungsstange 9 angeordnet sein.

Der zur Aufnahme der Zug-/Schubstange 4 dienende hohle Schaft 3 ist über einen Kopplungs- oder Rastmechanismus mit der Handhabe 2 koppelbar, der im Gehäuse 7 der Handhabe 2 angeordnet ist. Bei der dargestellten Ausführungsform des medizinischen Rohrschaftinstruments weist der hohle Schaft 3 weiterhin einen Spülanschluss 13 auf, der einerseits zum Einleiten von Spülflüssigkeit während einer Operation dient und an den andererseits zum Reinigen des hohlen Schafts 3 ein Spülschlauch anschließbar ist.

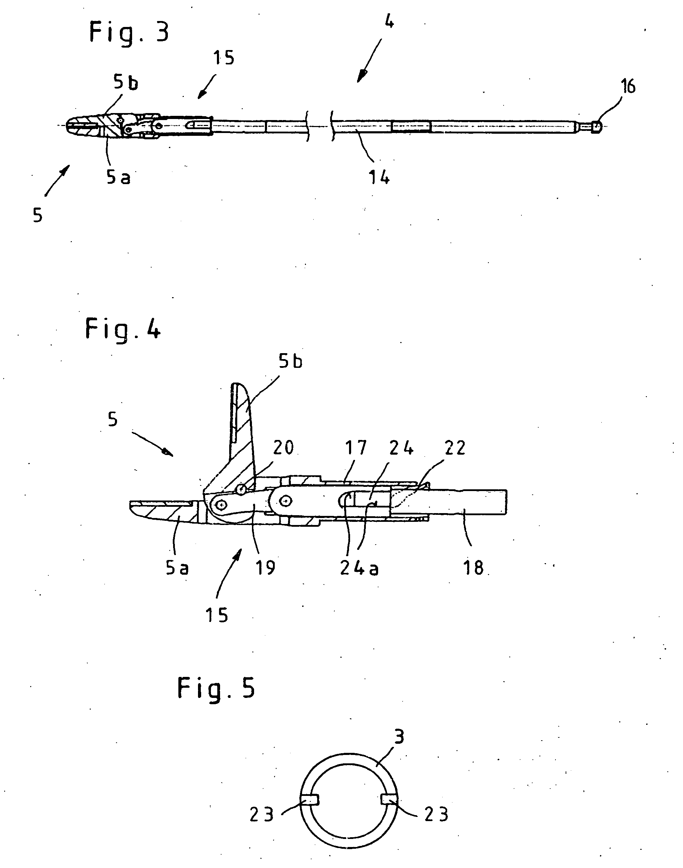

Der Aufbau der Zug-/Schubstange 4 sowie des Kopplungsmechanismus zum lösbaren Verbinden der Zug-/Schubstange 4 mit dem hohlen Schaft 3 ist insbesondere den Abbildungen Fig. 3 bis 6c und 8a bis 9c zu entnehmen.

Wie insbesondere aus Fig. 3 und 4 ersichtlich, besteht die Zug-/Schubstange 4 aus einem Stangengrundkörper 14 sowie einem das distale Ende der Zug-/Schubstange 4 bildenden Werkzeugeinsatz 15, wobei der das verschwenkbare Maulteil 5b tragende Werkzeugeinsatz 15 beispielsweise durch Verschweißen, Verschrauben oder Verkleben, fest mit dem Stangengrundkörper 14 verbindbar ist. Am proximalen Ende weist die Zug-/Schubstange 4 ein Kopplungselement 16 auf, welches zum Verbinden der Zug-/Schubstange 4 mit der Handhabe 2 dient.

Der Aufbau des Werkzeugeinsatzes 15 ist insbesondere den Abbildungen Fig. 4 und 6a bis 6c sowie 8a bis 9c zu entnehmen.

Bei der in den Abbildungen Fig. 4 und 6a bis 6c dargestellten ersten Ausführungsform besteht der Werkzeugeinsatz 15 aus einer Hülse 17, die einstückig mit einem als starres Maulteil 5a ausgestalteten Maulteil des Werkzeugs 5 ausgebildet ist. In der Hülse 17 ist in Axialrichtung verschiebbar ein Werkzeugschaft 18 gelagert, der distalsetig über einen Gelenkhebel 19 mit dem verschwenkbaren Maulteil 5b des Werkzeugs 5 verbunden ist und proximalseitig fest mit dem Stangengrundkörper 14 verbindbar ist. Um die Axialverschiebung der Zug-/Schubstange 4 über den Werkzeugschaft 18 direkt und weitestgehend spielfrei auf das verschwenkbare Maulteil 5b übertragen zu können, ist der Gelenkhebel 19 sowohl am verschwenkbaren Maulteil 5b als auch am Werkzeugschaft 18 verschwenkbar gelagert. Die mit dem starren Maulteil 5a versehene Hülse 17 und der Werkzeugschaft 18 sind über das veschwenkbare Maulteil 5b und eine starre Schwenkachse 20 des verschwenkbaren Maulteils 5b miteinander verbunden bzw. gekoppelt, wobei die Schwenkachse 20 in der Hülse 17 gelagert ist.

Alternativ zu der dargestellten Ausführungsform ist es selbstverständlich auch möglich, das Werkzeug 5 mit zwei zueinander verschwenkbaren Maulteilen 5b auszustatten.

Den Abbildungen Fig. 4 bis 6c ist schließlich der Aufbau und die Funktionsweise des Kopplungsmechanismus dieser ersten Ausführungsform zu entnehmen, über den die Zug-/Schubstange 4 und der hohle Schaft 3 lösbar miteinander verbindbar sind.

Dieser Kopplungsmechanismus ist als Zapfen-Schlitz-Steuerung 21 ausgebildet, die bei der dargestellten Ausführungsform aus zwei in der Hülse 17 des Werkzeugeinsatzes 15 ausgebildeten wendelfömigen Führungsbahnen 22 sowie zwei an der distalen Innenseite des hohlen Schaftes 3 ausgebildeten Steuerzapfen 23 zur Aufnahme in den Führungsbahnen 22 besteht.

Wie aus Fig. 5 ersichtlich, sind bei der dargestellten Ausgestaltungsform an der distalen Innenseite des hohlen Schaftes 3 zwei einander gegenüberliegend angeordnete Steuerzapfen 23 ausgebildet. Die Ausbildung von zwei Steuerzapfen 23 und zwei entsprechenden, einander gegenüberliegend angeordneten Führungsbahnen 22 hat den Vorteil, dass die beiden miteinander zu koppelnden Bauteile, der hohle Schaft 3 und die Zug-/Schubstange 4, nicht nur in einer einzigen Stellung der beiden Bauteile zueinander miteinander koppelbar sind, wodurch die Montage vereinfacht wird.

Um eine Selbsthemmung der Steuerzapfen 23 beim Eindrehen oder Herausdrehen aus den Führungsbahnen 22 zu vermeiden, sind die Führungsbahnen 22 vorzugsweise wendelförmig ausgebildet und weisen einen Steigungswinkel α auf, der in Abhängigkeit von der Reibungspaarung eine vorbestimmte maximale Steigung von vorzugsweise 45° nicht übersteigen soll.

Alternativ zu der dargestellten Ausführungsform ist es selbstverständlich auch möglich, die Zapfen-Schlitz-Steuerung 21 so auszubilden, dass der mindestens eine Steuerzapfen 23 am Werkzeugeinsatz 15 der Zug-/Schubstange 4 und die Führungsbahnen 22 zur Aufnahme des Steuerzapfens 23 am distalseitigen Ende des hohlen Schaftes 3 angeordnet sind.

Weiterhin ist im Werkzeugschaft 18 eine in Axialrichtung des Werkzeugschaftes 18 verlaufende proximalseitig offene Ausnehmung 24 zur verriegelnden Aufnahme der Steuerzapfen 23 in der zusammengesetzten Arbeitsstellung des Rohrschaftinstruments ausgebildet. Mindestens eine der in Axialrichtung des Werkzeugschaftes 18 im Wesentlichen parallel zueinander verlaufenden Kanten 24a der Ausnehmung 24 bilden in der Arbeitsstellung des Werkzeugs 5 eine begrenzende Anschlagfläche für jeden Steuerzapfen 23 und verhindern so ein selbstständiges Ausdrehen aus der zugehörigen Führungsbahn 22.

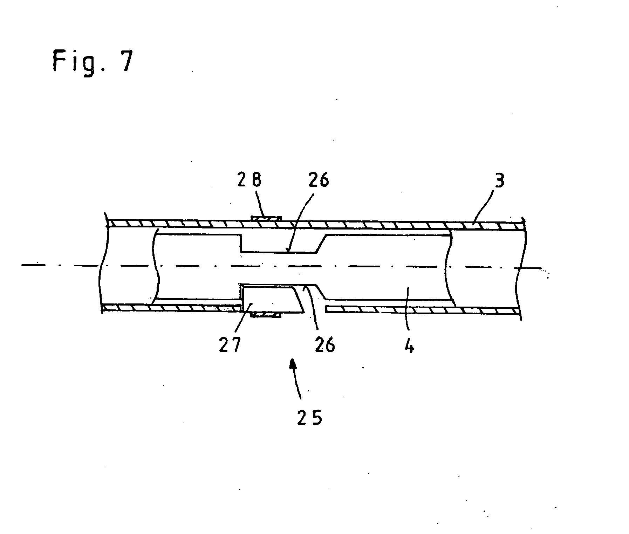

Zusätzlich zu dieser zuvor beschriebenen Sicherung des Kopplungsmechanismus, die ein versehentliches Überführen des Werkzeugs 5 in die über-offene Montagestellung verhindern soll, weist das dargestellte Rohrschaftinstrument im Bereich des in der Handhabe 2 gelagerten proximalen Endes der Zug-/Schubstange 4 und des hohlen Schaftes 3 eine die Verlagerbarkeit der Zug-/Schubstange 4 innerhalb des hohlen Schaftes 3 limitierende Begrenzungsvorrichtung 25 auf, wie diese schematisch in Fig. 7 dargestellt ist.

Auch die Begrenzungsvorrichtung 25 ist dazu ausgelegt, ein unbeabsichtigtes Entkoppeln des Werkzeugbetätigungselements 4 bzw. der Zug-/Schubstange 4 und des hohlen Schaftes 3 durch Überführen des Werkzeugs 5 in die über-offene Montagestellung zu verhindern.

Wie aus Fig. 7 ersichtlich, besteht die Begrenzungsvorrichtung 25 bei dem dargestellten Ausführungsbeispiel aus zwei am Werkzeugbetätigungselement 4 ausgebildeten, einander gegenüberliegend angeordneten Anlageflächen 26, die als Ausnehmungen im Werkzeugbetätigungselement 4 ausgebildet sind, sowie aus einem in einer Ausnehmung im hohlen Schaft 3 gelagerten Anlageelement 27, das im montierten Zustand von Werkzeugbetätigungselement 4 und hohlem Schaft 3 im Wesentlichen formschlüssig an einer der Anlageflächen 26 des Werkzeugbetätigungselements 4 anliegt.

In der in Fig. 7 dargestellten Arbeitstellung der Begrenzungsvorrichtung 25, die auch der Stellung entspricht, die die Begrenzungsvorrichtung 25 einnimmt, wenn der hohle Schaft 3 mit eingesetztem Werkzeugbetätigungselement 4 (Zug-/Schubstange 4) mit der Handhabe 2 gekoppelt sind, liegt das Anlageelement 27 einerseits formschlüssig an einer der Anlageflächen 26 an und schließt andererseits bündig mit der Mantelfläche des hohlen Schaftes 3 ab, so dass das Einführen des hohlen Schaftes 3 in die Handhabe 2 problemlos erfolgen kann. Da die axiale Erstreckung der Anlageflächen 26 die axiale Länge des Anlageelements 27 übersteigt ist es möglich, das Werkzeugbetätigungselement 4 (Zug-/Schubstange 4) um diese Längendifferenz in Axialrichtung innerhalb des hohlen Schaftes 3 zu verschieben. Dieser mögliche Axialhub des Werkzeugbetätigungselements 4 (Zug-/Schubstange 4) entspricht genau der Axialverschiebung des Werkzeugbetätigungselements 4 (Zug-/Schubstange 4) die notwendig ist, um das Werkzeug 5 von der geschlossenen in die geöffnete Arbeitsstellung und wieder zurück zu überführen.

Das Überführen des Werkzeugs in die zum Entkoppeln von Werkzeugbetätigungselement 4 (Zug-/Schubstange 4) und hohlem Schaft 3 notwendige über-offene Montagestellung des Werkzeugs 5 wird jedoch durch das Anlageelement 27 verhindert, da dieses einerseits einen direkten Anschlag für das Werkzeugbetätigungselement 4 (Zug-/Schubstange 4) bei einer weiteren Axialverschiebung bildet und andererseits eine Radialbewegung des Anlageelements 27 nach außen durch die Einbindung in die Handhabe 2 verhindert wird.

Zusätzlich zur Beschränkung des Axialhubs des Werkzeugbetätigungselements 4 (Zug-/Schubstange 4) dient die Begrenzungsvorrichtung 25 bei der dargestellten Ausgestaltung des Werkzeugbetätigungselements 4 als Zug-/Schubstange 4 zur Verhinderung einer Torsion der Zug-/Schubstange 4 um ihre Längsachse.

Sobald der hohle Schaft 3 mit dem darin gelagerten Werkzeugbetätigungselement 4 (Zug-/Schubstange 4) wieder von der Handhabe 2 entkoppelt ist, ist es möglich, durch Ausüben einer Druckkraft auf das entkoppelte proximale Ende des Werkzeugbetätigungselements 4 (Zug-/Schubstange 4) dieses in axialer Richtung soweit nach distal zu verschieben, bis das Werkzeug 5 die in Fig. 6a dargestellte über-offene Montagestellung einnimmt. Da im von der Handhabe 2 entkoppelten Zustand das Anlageelement 27 in radialer Richtung nach außen gedrückt werden kann, sobald eine größere Druckkraft auf das entkoppelte proximale Ende des Werkzeugbetätigungselements 4 (Zug-/Schubstange 4) ausgeübt wird, tritt die Begrenzungsvorrichtung 25 im von der Handhabe 2 entkoppelten Zustand außer Kraft und ermöglicht so das Entkoppeln von hohlem Schaft 3 und Werkzeugbetätigungselement 4 (Zug-/Schubstange 4).

Um die Handhabung der Begrenzungsvorrichtung 25 zu erleichtern, wird das Anlageelement 27, wie aus Fig. 7 ersichtlich, über einen elastischen Gummiring 28 in der im hohlen Schaft 3 ausgebildeten Ausnehmung gehalten. Diese federelastische Lagerung des Anlageelements 27 erlaubt einerseits weiterhin das radial nach außen gerichtete Austreten des Anlageelements 27 zur Freigabe des Werkzeugbetätigungselements 4 (Zug-/Schubstange 4) und gewährleistet andererseits das lagegerechte Wiedereinsetzen des Anlageelements in die Ausnehmung des hohlen Schaftes 3, so dass die Begrenzungsvorrichtung 25 sofort wieder in einen betriebsbereiten Zustand überführt wird. In erster Linie dient der elastische Gummiring 28 aber dazu, ein Herausfallen des Anlageelements 27 aus der Ausnehmung des hohlen Schaftes 3 zu verhindern.

Die zuvor beschriebene Arbeitsweise der Begrenzungsvorrichtung 25 ist, unabhängig von den praktischen Vorteilen des Gummirings 28, vollkommen unabhängig von der Verwendung des Gummirings 28.

Zusätzlich zu den beiden zuvor beschriebenen Sicherungsmaßnahmen, die ein versehentliches Überführen des Werkzeugs 5 in die über-offene Montagestellung verhindern sollen, oder auch alternativ zu wenigstens einer dieser Sicherungsmaßnahmen ist es möglich, den Verschwenkwinkel der Griffteile 1 der Handhabe 2 zueinander konstruktiv so zu beschränken, dass die Griffteile im fertig montierten Zustand des Rohrschaftinstruments nicht in eine Stellung überführbar sind, die das Überführen des Werkzeugs 5 in die über-offene Montagestellung bewirkt.

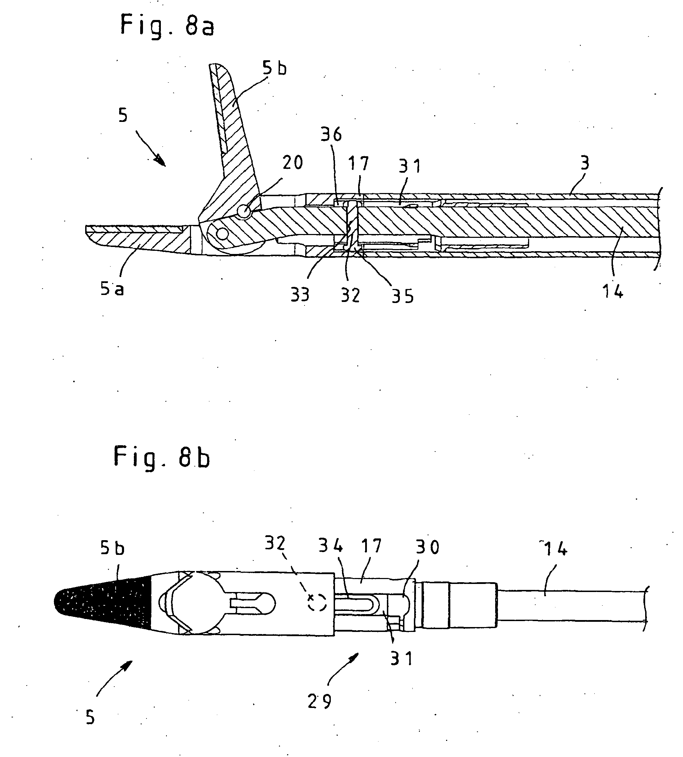

Bei der in den Abbildungen Fig. 8a bis 9c dargestellten zweiten Ausführungsform besteht der Werkzeugeinsatz 15 ebenfalls aus einer Hülse 17, die einstückig mit einem als starres Maulteil 5a ausgestalteten Maulteil des Werkzeugs 5 ausgebildet ist. In der Hülse 17 ist in Axialrichtung verschiebbar der Stangengrundkörper 14 der Zug-/Schubstange 4 gelagert, der distalsetig mit dem verschwenkbaren Maulteil 5b des Werkzeugs 5 verbunden ist. Um die Axialverschiebung der Zug-/Schubstange 4 direkt und weitestgehend spielfrei auf das verschwenkbare Maulteil 5b übertragen zu können, ist der Stangengrundkörper 14 verschwenkbar am verschwenkbaren Maulteil 5b gelagert. Die mit dem starren Maulteil 5a versehene Hülse 17 und der Stangengrundkörper 14 sind über das veschwenkbare Maulteil 5b und eine starre Schwenkachse 20 des verschwenkbaren Maulteils 5b miteinander verbunden bzw. gekoppelt, wobei die Schwenkachse 20 in der Hülse 17 gelagert ist.

Den Abbildungen Fig. 8 bis 9c ist schließlich der Aufbau und die Funktionsweise des Kopplungsmechanismus gemäß dieser zweiten Ausführungsform zu entnehmen, über den die Zug-/Schubstange 4 und der hohle Schaft 3 lösbar miteinander verbindbar sind.

Dieser Kopplungsmechanismus ist als Bajonettverbindung 29 ausgebildet, die aus mindestens einem in der Hülse 17 des Werkzeugeinsatzes 15 ausgebildeten Langloch 30 sowie mindestens einem an der distalen Innenseite des hohlen Schaftes 3 ausgebildeten Steuerzapfen in der Art der in Fig. 5 dargestellten Steuerzapfen 23 zur Aufnahme im Langloch 30 besteht.

Weiterhin ist in der Hülse 17 hülsenförmiger Führungseinsatz 31 angeordnet, der das distalseitige Ende des Stangengrundkörpers 14 koaxial umgibt. Im Bereich seines in dem Führungseinsatz 31 gelagerten distalen Endes weist der Stangengrundkörper 14 einen quer zur Längsachse des Stangengrundkörpers 14 angeordneten Verriegelungsstift 32 auf, der frei beweglich so in einer Bohrung 33 des Stangengrundkörpers 14 gelagert ist, dass die freien Enden des Verriegelungsstiftes 32 beidseitig im Führungseinsatz 31 ausgebildete Führungsbahnen 34 durchragen. Die die Führungsbahnen 34 überragenden freien Enden des Verriegelungsstiftes 32 sind bei der dargestellten Ausführungsform als bolzenkopfförmige Verdickungen 35 ausgebildet, die das Herausrutschen des Verriegelungsstiftes 32 aus den Führungsbahnen 34 und der Bohrung 33 verhindern.

Das Zusammensetzen des dargestellten Rohrschaftinstruments und insbesondere des Kopplungsmechanismus zum Verbinden des hohlen Schaftes 3 mit der Zug-/Schubstange 4 wird nachfolgend anhand der Abbildungen Fig. 6a bis 6c sowie 8a bis 9c für beide Ausführungsbeispiele beschrieben.

### Ausführungsbeispiel gemäß Fig. 6a bis 6c

Zu Beginn der Montage wird das Werkzeug 5 durch Öffnen der Maulteile 5a und 5b in die in Fig. 6a dargestellte Montagestellung überführt, in der sich das verschwenkbare Maulteil 5b in einer extrem weit geöffneten Position befindet, in die das Maulteil 5b des zusammengesetzten Rohrschaftinstruments nicht überführbar ist.

In dieser Montagestellung wird nun der hohle Schaft 3 mit seinem distalseitigen Ende voran auf die Zug-/Schubstange 4 aufgeschoben, bis das distalseitige Ende gegen die Hülse 17 des Werkzeugeinsatzes 15 anläuft. Das Koppeln der Bauteile hohler Schaft 3 und Zug-/Schubstange 4 erfolgt dann durch Verdrehen der Bauteile gegeneinander, bis die Steuerzapfen 23 in die Führungsbahnen 22 eingreifen. Anschließend werden die Bauteile 3 und 4 so lange weiter gegeneinander verdreht, bis der Steuerzapfen 23 das Ende der Führungsbahn 22 erreicht hat.

Wenn nun die Zug-/Schubstange 4 in axialer Richtung hin zum proximalen Ende gezogen wird oder aber die Maulteile 5a, 5b zusammengedrückt werden, tritt der in der Führungsbahn 22 angeordnete Steuerzapfen 23 in die im Werkzeugschaft 18 ausgebildete Ausnehmung 24 ein.

Die Abbildungen Fig. 6b und 6c zeigen das Werkzeug 5 in den beiden extremen Arbeitspositionen der Maulteile, nämlich der geöffneten Arbeitsstellung gemäß Fig. 6b und der geschlossenen Arbeitsstellung gemäß Fig. 6c. Wie aus den zugehörigen Darstellungen ersichtlich, liegt der Steuerzapfen 23 in diesen Positionen an der oberen Kante 24a der Ausnehmung 24 an, so dass die Kante 24a eine begrenzende Anschlagfläche für den Steuerzapfen 23 bildet die verhindert, dass der Steuerzapfen 23 wieder aus der Führungsbahn 22 herausgedreht werden kann.

Dank dieser selbstsichernden Wirkung der Zapfen-Schlitz-Steuerung 21 besteht bei der nachfolgenden weiteren Montage des Rohrschaftinstruments nicht die Gefahr, dass sich die im ersten Montageschritt zusammengesetzten Bauteile hohler Schaft 3 und Zug-/Schubstange 4 wieder lösen.

In den nächsten Montageschritten werden dann der hohle Schaft 3 und die Handhabe 2 miteinander verrastet und die Zug-/Schubstange 4 mit den Griffteilen 1 der Handhabe 2 verbunden.

Die Demontage erfolgt dann in der genau umgekehrten Reihenfolge der Montageschritte über das Lösen der Verrastung zwischen dem hohlen Schaft 3 und der Handhabe 2, das Lösen der Verbindung der Zug-/Schubstange 4 mit den Griffteilen 1 der Handhabe 2 bis hin zum Entkoppeln des hohlen Schaftes 3 von der Zug-/Schubstange 4 durch Lösen der Zapfen-Schlitz-Steuerung 21. Hierzu ist es zunächst erforderlich, das Werkzeug 5 durch einen leichten Schlag oder Ausüben einer Druckkraft per Hand auf das entkoppelte proximale Ende der Zug-/Schubstange 4 in die Montagestellung zu überführen, um die Blockierung des Steuerzapfens 23 durch die Kante 24a der Ausnehmung 24 aufzuheben. Anschließend lässt sich der Steuerzapfen 23 einfach aus der Führungsbahn 22 herausdrehen, um den hohlen Schaft 3 nachfolgend von der Zug-/Schubstange 4 abziehen zu können.

### Ausführungsbeispiel gemäß Fig. 8a bis 9c

Zu Beginn der Montage wird das Werkzeug 5 durch Öffnen der Maulteile 5a und 5b in die in Fig. 8a dargestellte Montagestellung überführt, in der sich das verschwenkbare Maulteil 5b in einer extrem weit geöffneten Position befindet, in die das Maulteil 5b des zusammengesetzten Rohrschaftinstruments nicht überführbar ist.

In dieser Montagestellung wird die Zug-/Schubstange 4 so weit wie möglich in distaler Richtung geschoben, bis der quer zur Längsachse des Stangengrundkörpers 14 angeordnete Verriegelungsstift 32 eine auf der Innenseite der Hülse 17 ausgebildete Aufnahme 36 eintritt, wie dies in Fig. 8a dargestellt ist. In dieser überoffenen Montageposition des verschwenkbaren Maulteils 5b lassen sich nun über die Bajonettverbindung 29 der hohle Schaft 3 und die Zug-/Schubstange 4 miteinander verbinden.

Die Abbildungen Fig. 9a, 9b und 9c zeigen das Werkzeug 5 in den beiden extremen Arbeitspositionen der Maulteile, nämlich der geöffneten Arbeitsstellung gemäß Fig. 9a und 9b und der geschlossenen Arbeitsstellung gemäß Fig. 9c. Wie aus den zugehörigen Darstellungen ersichtlich, greift der Verriegelungsstift 32 in diesen Positionen in das Langloch 30 der Bajonettverbindung 29 ein und sichert so die Zug-/Schubstange 4 und somit auch die Maulteile 5a und 5b gegen Verdrehen, so dass die Bajonettverbindung 29 in den Arbeitspositionen nicht trennbar ist.

Die aus den Abbildungen Fig. 9a und 9c ersichtliche auf-und-ab Pendelbewegung der Zug-/Schubstange 4 beim Verstellen der Maulteile 5a und 5b zwischen der geschlossenen Arbeitsstellung (Fig. 9c) und der offenen Arbeitsstellung (Fig. 9a und 9b) hat keinen Einfluss auf den verriegelnden Eingriff des Verriegelungsstiftes 32 in das Langloch 30, da der Verriegelungsstift 32 frei beweglich in der Bohrung 33 des Stangengrundkörpers 14 gelagert ist.

In den nächsten Montageschritten werden dann der hohle Schaft 3 und die Handhabe 2 miteinander verrastet und die Zug-/Schubstange 4 mit den Griffteilen 1 der Handhabe 2 verbunden.

Die Demontage erfolgt dann in der genau umgekehrten Reihenfolge der Montageschritte über das Lösen der Verrastung zwischen dem hohlen Schaft 3 und der Handhabe 2, das Lösen der Verbindung der Zug-/Schubstange 4 mit den Griffteilen 1 der Handhabe 2 bis hin zum Entkoppeln des hohlen Schaftes 3 von der Zug-/Schubstange 4 durch Lösen der Bajonettverbindung 29.

### Bezugszeichenliste

- 1: Griffteil
- 2: Handhabe
- 3: hohler Schaft
- 4: Werkzeugbetätigungselement/Zug-/Schubstange
- 5: Werkzeug
- 5a: starres Maulteil
- 5b: verschwenkbares Maulteil
- 6: Anlenkpunkt
- 7: Gehäuse
- 8: Gelenkhebel
- 9: Kupplungsstange
- 10: Kupplungsgehäuse
- 11: Arretiervorrichtung
- 12: Entriegelungsknopf
- 13: Spülanschluss
- 14: Stangengrundkörper

- 15: Werkzeugeinsatz
- 16: Kopplungselement
- 17: Hülse
- 18: Werkzeugschaft

- 19: Gelenkhebel
- 20: Schwenkachse
- 21: Zapfen-Schlitz-Steuerung
- 22: Führungsbahn
- 23: Steuerzapfen
- 24: Ausnehmung
- 24a: Kante
- 25: Begrenzungsvorrichtung
- 26: Anlagefläche
- 27: Anlageelement
- 28: Gummiring
- 29: Bajonettverbindung
- 30: Langloch
- 31: Führungseinsatz
- 32: Verriegelungsstift
- 33: Bohrung
- 34: Führungsbahn
- 35: Verdickung
- 36: Aufnahme

- α: Steigungswinkel

## Patentansprüche

1. Medizinisches Rohrschaftinstrument mit einem hohlen Schaft (3), einer am proximalem Ende des Schaftes (3) angeordneten Handhabe (2) und mindestens einem in dem hohlen Schaft (3) gelagerten Werkzeugbetätigungselement (4), an dessen distalem Ende ein aus zwei Maulteilen (5a, 5b) bestehendes Werkzeug (5) angeordnet ist, wobei das Werkzeugbetätigungselement (4) zum Betätigen des Werkzeugs (5) mit mindestens einem Betätigungsmechanismus der Handhabe (2) koppelbar ist und wobei das Werkzeugbetätigungselement (4) und der hohle Schaft (3) über einen Kopplungsmechanismus lösbar miteinander verbindbar sind,
**dadurch gekennzeichnet,**
**dass** die Arretierung des Kopplungsmechanismus zwischen dem Werkzeugbetätigungselement (4) und dem hohlen Schaft (3) in Abhängigkeit von der Stellung der Maulteile (5a, 5b) des Werkzeugs (5) zueinander erfolgt, wobei das Werkzeugbetätigungselement (4) ausschließlich in einer von den Arbeitspositionen abweichenden, über-offenen Montagestellung der Maulteile (5a,5b) über den Kopplungsmechanismus mit dem hohlen Schaft (3) verbindbar ist.

2. Medizinisches Rohrschaftinstrument nach Anspruch 1, **dadurch gekennzeichnet, dass** der Kopplungsmechanismus in der Art einer Bajonettverbindung ausgebildet ist.

3. Medizinisches Rohrschaftinstrument nach Anspruch 1, **dadurch gekennzeichnet, dass** der Kopplungsmechanismus als selbstsichernde Zapfen-Schlitz-Steuerung (21) ausgebildet ist, die aus mindestens einem an einem der miteinander zu koppelnden Bauteile (3, 4) ausgebildeten Steuerzapfen (23) und mindestens einer am jeweils anderen Bauteil (4, 3) ausgebildeten Führungsbahn (22) zur Aufnahme eines Steuerzapfens (23) besteht und dass mindestens ein in eine Führungsbahn (22) eingesetzter Steuerzapfen (23) in der Arbeitsposition des Werkzeugs (5) gegen Entnahme aus der Führungsbahn (22) blockiert ist.

4. Medizinisches Rohrschaftinstrument nach Anspruch 3, **dadurch gekennzeichnet, dass** der mindestens eine Steuerzapfen (23) distalseitig an der Innenseite des hohlen Schaftes (3) ausgebildet ist.

5. Medizinisches Rohrschaftinstrument nach Anspruch 4, **dadurch gekennzeichnet, dass** an der Innenseite des distalseitigen Ende des hohlen Schaftes (3) ein Steuerzapfen (23) ausgebildet ist und am distalseitigen Ende des Werkzeugbetätigungselements (4) zwei einander gegenüberliegende Führungsbahnen (22) zur Aufnahme des mindestens einen Steuerzapfens (23) ausgebildet sind.

6. Medizinisches Rohrschaftinstrument nach einem der Ansprüche 1 bis 5, **dadurch gekennzeichnet, dass** der Betätigungsmechanismus der Handhabe (2) als verschwenkbar an der Handhabe (2) gelagerter Griffteil (1) ausgebildet ist.

7. Medizinisches Rohrschaftinstrument nach einem der Ansprüche 1 bis 6, **dadurch gekennzeichnet, dass** das Werkzeug (5) als aus mindestens zwei Maulteilen (5a, 5b) bestehendes Werkzeug (5) ausgebildet ist, wobei mindestens ein Maulteil (5b) des Werkzeugs (5) über das Werkzeugbetätigungselement (4) gegenüber dem anderen Maulteil (5a) verschwenkbar ist.

8. Medizinisches Rohrschaftinstrument nach einem der Ansprüche 1 bis 7, **dadurch gekennzeichnet, dass** das Werkzeugbetätigungselement (4) als Zug-Schubstange (4) ausgebildet ist.

9. Medizinisches Rohrschaftinstrument nach Anspruch 8, **dadurch gekennzeichnet, dass** die Zug-/Schubstange (4) aus einem Stangengrundkörper (14) und einem mit dem Stangengrundkörper (14) fest verbindbaren, das distale Ende der Zug-/Schubstange (4) bildenden Werkzeugeinsatz (15) besteht.

10. Medizinisches Rohrschaftinstrument nach Anspruch 9, **dadurch gekennzeichnet, dass** der Werkzeugeinsatz (15) aus einer mit einem starren Maulteil (5a) des Werkzeugs (5) versehenen Hülse (17) und einem verschiebbar in der Hülse (17) gelagerten Werkzeugschaft (18) besteht, der einerseits mit einem verschwenkbaren Maulteil (5b) des Werkzeugs (5) gekoppelt ist und andererseits fest mit dem Stangengrundkörper (14) verbindbar ist.

11. Medizinisches Rohrschaftinstrument nach Anspruch 3 und 10, **dadurch gekennzeichnet, dass** im Werkzeugschaft (18) des Werkzeugeinsatzes (15) eine in Axialrichtung des Werkzeugschaftes (18) verlaufende proximalseitig offene Ausnehmung (24) zur Aufnahme des Steuerzapfens (23) ausgebildet ist.

12. Medizinisches Rohrschaftinstrument nach Anspruch 11, **dadurch gekennzeichnet, dass** mindestens eine der in Axialrichtung des Werkzeugschaftes (18) im Wesentlichen parallel zueinander verlaufenden Kanten (24a) der Ausnehmung (24) in der Arbeitsstellung des Werkzeugs (5) eine begrenzende Anschlagfläche für den Steuerzapfen (23) bildet.

13. Medizinisches Rohrschaftinstrument nach einem der Ansprüche 11 bis 12, **dadurch gekennzeichnet, dass** die Führungsbahn (22) zur Aufnahme eines Steuerzapfens (23) in der Hülse (17) des Werkzeugeinsatzes (15) ausgebildet ist.

14. Medizinisches Rohrschaftinstrument nach Anspruch 13, **dadurch gekennzeichnet, dass** die Führungsbahn (22) wendelförmig ausgebildet ist.

15. Medizinisches Rohrschaftinstrument nach Anspruch 14, **dadurch gekennzeichnet, dass** die Führungsbahn (22) einen Steigungswinkel a aufweist, der in Abhängigkeit von der Reibungspaarung von Steuerzapfen (23) und Führungsbahn (22) eine vorbestimmte maximale Steigung, vorzugsweise 45°, nicht übersteigt.

16. Medizinisches Rohrschaftinstrument nach einem der Ansprüche 1 bis 15, **dadurch gekennzeichnet, dass** im Bereich des in der Handhabe (2) gelagerten proximalen Endes des Werkzeugbetätigungselements (4) und des hohlen Schaftes (3) eine die Verlagerbarkeit des Werkzeugbetätigungselements (4) innerhalb des hohlen Schaftes (3) limitierende Begrenzungsvorrichtung (25) angeordnet ist.

17. Medizinisches Rohrschaftinstrument nach Anspruch 16, **dadurch gekennzeichnet, dass** die Begrenzungsvorrichtung (25) aus mindestens einer am Werkzeugbetätigungselement (4) ausgebildeten Anlagefläche (26) und einem mit dieser Anlagefläche (26) in Wirkverbindung bringbaren, im hohlen Schaft (3) gelagerten Anlageelement (27) besteht.

18. Medizinisches Rohrschaftinstrument nach Anspruch 16, **dadurch gekennzeichnet, dass** am Werkzeugbetätigungselement (4) zwei einander gegenüberliegende, als Ausnehmungen ausgebildete Anlageflächen (26) angeordnet sind und in einer Ausnehmung im hohlen Schaft (3) ein Anlageelement (27) gelagert ist, das im montierten Zustand von Werkzeugbetätigungselement (4) und hohlem Schaft (3) im Wesentlichen formschlüssig an einer der Anlageflächen (26) des Werkzeugbetätigungselements (4) anliegt.

19. Medizinisches Rohrschaftinstrument nach einem der Ansprüche 10 bis 15, **dadurch gekennzeichnet, dass** der Werkzeugschaft (18) und das verschwenkbare Maulteil (5b) über mindestens einen beidseitig verschwenkbar gelagerten Gelenkhebel (19) miteinander verbunden sind.

20. Medizinisches Rohrschaftinstrument nach einem der Ansprüche 1 bis 19, **dadurch gekennzeichnet, dass** das Entkoppeln des Werkzeugbetätigungselements (4) vom hohlen Schaft (3) über einen auf das proximale Ende des Werkzeugbetätigungselements (4) ausgeübten Schlag erfolgt, der das Werkzeug (5) in die Montagestellung überführt.

21. Medizinisches Rohrschaftinstrument nach Anspruch 1 oder 2, **dadurch gekennzeichnet, dass** das als Zug-Schubstange (4) ausgebildete Werkzeugbetätigungselement (4) aus einem Stangengrundkörper (14) und einem mit dem Stangengrundkörper (14) fest verbindbaren, das distale Ende der Zug-/Schubstange (4) bildenden Werkzeugeinsatz (15) besteht, wobei der Werkzeugeinsatz (15) aus einer mit einem starren Maulteil (5a) des Werkzeugs (5) versehenen Hülse (17) und einem in der Hülse (17) angeordneten, das distale Ende der Zug-/Schubstange (4) koaxial umgebenden Führungseinsatz (31) besteht.

22. Medizinisches Rohrschaftinstrument nach Anspruch 21, **dadurch gekennzeichnet, dass** die Zug-/Schubstange (4) im Bereich ihres in dem Führungseinsatz (31) gelagerten distalen Endes einen quer zur Längsachse der Zug-/Schubstange (4) angeordneten Verriegelungsstift (32) aufweist.

23. Medizinisches Rohrschaftinstrument nach Anspruch 22, **dadurch gekennzeichnet, dass** der Verriegelungsstift (32) frei beweglich so in einer Bohrung (33) der Zug-/Schubstange (4) gelagert ist, dass die freien Enden des Verriegelungsstiftes (32) im Führungseinsatz (31) ausgebildete Führungsbahnen (34) durchragen.

24. Medizinisches Rohrschaftinstrument nach Anspruch 22 oder 23, **dadurch gekennzeichnet, dass** die freien Enden des Verriegelungsstiftes (32) als bolzenkopfförmige Verdickungen (35) ausgebildet sind.

## Claims

1. A medical tube shaft instrument with a hollow shaft (3), a handle (2) arranged at the proximal end of the shaft (3) and at least one tool actuating element (4) which is mounted in the hollow shaft (3) and at the distal end of which, a tool (5) consisting of two jaw parts (5a, 5b) is arranged, wherein for actuating the tool (5), the tool actuating element (4) can be coupled with at least one actuating mechanism of the handle (2) and wherein the tool actuating element (4) and the hollow shaft (3) can be connected to each other in a detachable manner via a coupling mechanism,
**characterized in**
**that** the locking of the coupling mechanism between the tool actuating element (4) and the hollow shaft (3) takes place depending on the position of the jaw parts (5a, 5b) of the tool (5) relative to each other, wherein the tool actuating element (4) can be connected via the coupling mechanism to the hollow shaft (3) exclusively in a wide opened mounting position of the jaw parts (5a, 5b) which deviates from the working positions.

2. The medical tube shaft instrument according to claim 1, **characterized in that** the coupling mechanism is formed in the manner of a bayonet connection.

3. The medical tube shaft instrument according to claim 1, **characterized in that** the coupling mechanism is formed as self-locking pin-slot control (21) which consists of at least one control pin (23) formed on one of the components (3, 4) to be coupled with each other and at least one guideway (22) formed on the respective other component (4, 3) for receiving a control pin (23) and that at least one control pin (23) inserted in a guideway (22) is blocked in the working position of the tool (5) against removal from the guideway (22).

4. The medical tube shaft instrument according to claim 3, **characterized in that** the at least one control pin (23) is formed on the distal side on the inner surface of the hollow shaft (3).

5. The medical tube shaft instrument according to claim 4, **characterized in that** on the inner surface of the distal end of the hollow shaft (3), a control pin (23) is formed and at the distal end of the tool actuating element (4), two oppositely located guideways (22) for receiving the at least one control pin (23) are formed.

6. The medical tube shaft instrument according to any one of the claims 1 to 5, **characterized in that** the actuating mechanism of the handle (2) is formed as gripping part (1) pivotably mounted on the handle (2).

7. The medical tube shaft instrument according to any one of the claims 1 to 6, **characterized in that** the tool (5) is formed as tool (5) consisting of at least two jaw parts (5a, 5b), wherein at least one jaw part (5b) of the tool (5) can be pivoted relative to the other jaw part (5a) via the tool actuating element (4).

8. The medical tube shaft instrument according to any one of the claims 1 to 7, **characterized in that** the tool actuating element (4) is formed as pull-push rod (4).

9. The medical tube shaft instrument according to claim 8, **characterized in that** the pull/push rod (4) consists of a rod base body (14) and a tool insert (15) which can be fixedly connected to the rod base body (14) and forms the distal end of the pull/push rod (4).

10. The medical tube shaft instrument according to claim 9, **characterized in that** the tool insert (15) consists of a sleeve (17) provided with rigid jaw part (5a) of the tool (5) and a tool shaft (18) displaceably mounted in the sleeve (17), which tool shaft is coupled on one side with a pivotable jaw part (5b) of the tool (5) and can be fixedly connected on the other side to the rod base body (14).

11. The medical tube shaft instrument according to claim 3 and claim 10, **characterized in that** in the tool shaft (18) of the tool insert (15), a recess (24) extending in the axial direction of the tool shaft (18) and open on the proximal side is formed for receiving the control pin (23).

12. The medical tube shaft instrument according to claim 11, **characterized in that** at least one of the recess (24) edges (24a) extending substantially parallel to each other in the axial direction of the tool shaft (18) forms in the working position of the tool (5) a limiting stop surface for the control pin (23).

13. The medical tube shaft instrument according to any one of the claims 11 to 12, **characterized in that** the guideway (22) for receiving the control pin (23) is formed in the sleeve (17) of the tool insert (15).

14. The medical tube shaft instrument according to claim 13, **characterized in that** the guideway (22) is helically formed.

15. The medical tube shaft instrument according to claim 14, **characterized in that** the guideway (22) has a lead angle α which depending on the friction pairing of control pin (23) and guideway (22) does not exceed a predetermined maximum lead, preferably 45°.

16. The medical tube shaft instrument according to any one of the claims 1 to 15, **characterized in that** in the region of the proximal end of the tool actuating element (4) mounted in the handle (2) and the hollow shaft (3), a limiter device (25) limiting the displaceability of the tool actuating element (4) within the hollow shaft (3) is arranged.

17. The medical tube shaft instrument according to claim 16, **characterized in that** the limiter device (25) consists of at least one support face (26) formed on the tool actuating element (4) and a support element (27) which is mounted in the hollow shaft (3) and can be operatively connected to said support face (26).

18. The medical tube shaft instrument according to claim 16, **characterized in that** two support faces (26) located opposite each other and formed as recesses are arranged on the tool actuating element (4), and a support element (27) is mounted in a recess in the hollow shaft (3), which support element, in the assembled state of tool actuating element (4) and hollow shaft (3), rests in a substantially form-fitting manner against one of the support faces (26) of the tool actuating element (4).

19. The medical tube shaft instrument according to any one of the claims 10 to 15, **characterized in that** the tool shaft (18) and the pivotable jaw part (5b) are connected to each other via at least one articulated lever (19) which is pivotably mounted on both sides.

20. The medical tube shaft instrument according to any one of the claims 1 to 19, **characterized in that** decoupling the tool actuating element (4) from the hollow shaft (3) is carried out via a strike against the proximal end of the tool actuating element (4), which strike transfers the tool (5) into the mounting position.

21. The medical tube shaft instrument according to claim 1 or claim 2, **characterized in that** the tool actuating element (4) formed as pull/push rod (4) consists of a rod base body (14) and a tool insert (15) which can be fixedly connected to the rod base body (14) and forms the distal end of the pull/push rod (4), wherein the tool insert (15) consists of a sleeve (17) provided with a rigid jaw part (5a) of the tool (5) and a guide insert (31) which is arranged in the sleeve (17) and coaxially surrounds the distal end of the pull/push rod (4).

22. The medical tube shaft instrument according to claim 21, **characterized in that** in the region of its distal end mounted in the guide insert (31), the pull/push rod (4) has a locking pin (32) arranged transverse to the longitudinal axis of the pull/push rod (4).

23. The medical tube shaft instrument according to claim 22, **characterized in that** the locking pin (32) is mounted freely movable in such a manner in a bore (33) of the pull/push rod (4) that the free ends of the locking pin (32) protrude through guideways (34) formed in the guide insert (31).

24. The medical tube shaft instrument according to claim 22 or 23, **characterized in that** the free ends of the locking pin (32) are formed as bolt head-shaped thickenings (35).

## Revendications

1. Instrument médical à corps allongé tubulaire comprenant un corps allongé creux (3), une poignée de manoeuvre (2) agencée à l'extrémité proximale du corps allongé creux (3), et au moins un élément d'actionnement d'outil (4) monté dans le corps allongé creux (3) et à l'extrémité distale duquel est agencé un outil (5) constitué de deux pièces de mâchoire (5a, 5b), l'élément d'actionnement d'outil (4) pouvant, pour l'actionnement de l'outil (5), être couplé à au moins un mécanisme d'actionnement de la poignée de manoeuvre (2), et l'élément d'actionnement d'outil (4) et le corps allongé creux (3) pouvant être reliés l'un à l'autre de manière amovible par l'intermédiaire d'un mécanisme de couplage, **caractérisé en ce que** le blocage du mécanisme de couplage entre l'élément d'actionnement d'outil (4) et le corps allongé creux (3) s'effectue en fonction de la position des pièces de mâchoire (5a, 5b) de l'outil (5) l'une par rapport à l'autre, l'élément d'actionnement d'outil (4) pouvant être relié par l'intermédiaire du mécanisme de couplage au corps allongé creux (3), uniquement dans une position de montage des pièces de mâchoire (5a, 5b), qui diffère des positions de travail et correspond à une ouverture excessive.

2. Instrument médical à corps allongé tubulaire selon la revendication 1, **caractérisé en ce que** le mécanisme de couplage est réalisé à la manière d'une liaison du type à baïonnette.

3. Instrument médical à corps allongé tubulaire selon la revendication 1, **caractérisé en ce que** le mécanisme de couplage est réalisé sous la forme d'une commande à tenon et fente (21) auto-assurée, qui est constituée d'au moins un tenon de commande (23) réalisé sur l'une des pièces (3, 4) à coupler l'une à l'autre, et d'au moins une voie de guidage (22) réalisée respectivement sur l'autre pièce (4, 3) et destinée à recevoir un tenon de commande (23), et **en ce qu'**au moins un tenon de commande (23) inséré dans une voie de guidage (22) est, dans la position de travail de l'outil (5), bloqué à l'encontre d'un retrait hors de la voie de guidage (22).

4. Instrument médical à corps allongé tubulaire selon la revendication 3, **caractérisé en ce que** ledit au moins un tenon de commande (23) est réalisé du côté distal, sur le côté intérieur du corps allongé creux (3).

5. Instrument médical à corps allongé tubulaire selon la revendication 4, **caractérisé en ce que** sur le côté intérieur de l'extrémité côté distal du corps allongé creux (3), est réalisé un tenon de commande (23), et à l'extrémité côté distal de l'élément d'actionnement d'outil (4), sont réalisées deux voies de guidage (22) mutuellement opposées, pour recevoir ledit au moins un tenon de commande (23).

6. Instrument médical à corps allongé tubulaire selon l'une des revendications 1 à 5, **caractérisé en ce que** le mécanisme d'actionnement de la poignée de manoeuvre (2) est réalisé en tant que branche de préhension (1) montée pivotante sur la poignée de manoeuvre (2).

7. Instrument médical à corps allongé tubulaire selon l'une des revendications 1 à 6, **caractérisé en ce que** l'outil (5) est réalisé en tant qu'outil (5) constitué d'au moins deux pièces de mâchoire (5a, 5b), au moins une pièce de mâchoire (5b) de l'outil (5) étant susceptible de pivoter par rapport à l'autre pièce de mâchoire (5a) par l'intermédiaire de l'élément d'actionnement d'outil (4).

8. Instrument médical à corps allongé tubulaire selon l'une des revendications 1 à 7, **caractérisé en ce que** l'élément d'actionnement d'outil (4) est réalisé en tant que tige de traction/poussée (4).

9. Instrument médical à corps allongé tubulaire selon la revendication 8, **caractérisé en ce que** la tige de traction/poussée (4) est constituée d'un corps de base de tige (14) et d'un insert d'outil (15) pouvant être relié de manière fixe au corps de base de tige (14) et formant l'extrémité distale de la tige de traction/poussée (4).

10. Instrument médical à corps allongé tubulaire selon la revendication 9, **caractérisé en ce que** l'insert d'outil (15) est constitué d'une douille (17) reliée à une pièce de mâchoire fixe (5a) de l'outil (5), et d'une queue d'outil (18) qui est montée coulissante dans la douille (17), et qui d'une part est couplée à une pièce de mâchoire pivotante (5b) de l'outil (5), et d'autre part peut être reliée de manière fixe au corps de base de tige (14).

11. Instrument médical à corps allongé tubulaire selon les revendications 3 et 10, **caractérisé en ce que** dans la queue d'outil (18) de l'insert d'outil (15) est réalisé un évidement (24) s'étendant dans la direction axiale de la queue d'outil (18), ouvert du côté proximal et destiné à recevoir le tenon de commande (23).

12. Instrument médical à corps allongé tubulaire selon la revendication 11, **caractérisé en ce qu'**au moins un des bords (24a) de l'évidement (24), qui s'étendent sensiblement parallèlement les uns aux autres dans la direction axiale de la queue d'outil (18), forme, dans la position de travail de l'outil (5), une surface de butée de limitation pour le tenon de commande (23).

13. Instrument médical à corps allongé tubulaire selon l'une des revendications 11 à 12, **caractérisé en ce que** la voie de guidage (22) destinée à recevoir un tenon de commande (23), est réalisée dans la douille (17) de l'insert d'outil (15).

14. Instrument médical à corps allongé tubulaire selon la revendication 13, **caractérisé en ce que** la voie de guidage (22) est d'une configuration en forme d'hélice.

15. Instrument médical à corps allongé tubulaire selon la revendication 14, **caractérisé en ce que** la voie de guidage (22) présente un angle d'inclinaison d'hélice α qui, en fonction du couple de friction formé par le tenon de commande (23) et la voie de guidage (22), ne dépasse pas une inclinaison d'hélice maximale prédéterminée, de préférence de 45°.

16. Instrument médical à corps allongé tubulaire selon l'une des revendications 1 à 15, **caractérisé en ce que** dans la zone de l'extrémité proximale de l'élément d'actionnement d'outil (4), montée dans la poignée de manoeuvre (2), et du corps allongé creux (3), est agencé un dispositif de limitation (25) qui limite la possibilité de déplacement de l'élément d'actionnement d'outil (4) à l'intérieur du corps allongé creux (3).

17. Instrument médical à corps allongé tubulaire selon la revendication 16, **caractérisé en ce que** le dispositif de limitation (25) est constitué par au moins une surface d'appui (26) formée sur l'élément d'actionnement d'outil (4), et par un élément d'appui (27) monté dans le corps allongé creux (3) et pouvant être amené en interaction avec ladite surface d'appui (26).

18. Instrument médical à corps allongé tubulaire selon la revendication 16, **caractérisé en ce que** sur l'élément d'actionnement d'outil (4) sont agencées deux surfaces d'appui (26) mutuellement opposées réalisées sous la forme d'évidements, et dans un évidement du corps allongé creux (3) est monté un élément d'appui (27), qui, dans l'état monté de l'élément d'actionnement d'outil (4) et du corps allongé creux (3), s'appuie sensiblement par complémentarité de formes sur l'une des surfaces d'appui (26) de l'élément d'actionnement d'outil (4).

19. Instrument médical à corps allongé tubulaire selon l'une des revendications 10 à 15, **caractérisé en ce que** la queue d'outil (18) et la pièce de mâchoire pivotante (5b) sont mutuellement reliées par l'intermédiaire d'au moins un levier d'articulation (19) monté pivotant des deux côtés.

20. Instrument médical à corps allongé tubulaire selon l'une des revendications 1 à 19, **caractérisé en ce que** le découplage de l'élément d'actionnement d'outil (4) du corps allongé creux (3), s'effectue grâce à un choc exercé sur l'extrémité proximale de l'élément d'actionnement d'outil (4), qui transfère l'outil (5) dans la position de montage.

21. Instrument médical à corps allongé tubulaire selon la revendication 1 ou la revendication 2, **caractérisé en ce que** l'élément d'actionnement d'outil (4) réalisé sous forme de tige de traction/poussée (4) est constitué d'un corps de base de tige (14) et d'un insert d'outil (15) pouvant être relié de manière fixe au corps de base de tige (14) et formant l'extrémité distale de la tige de traction/poussée (4), l'insert d'outil (15) étant constitué d'une douille (17) reliée à une pièce de mâchoire fixe (5a) de l'outil (5), et d'un insert de guidage (31) agencé dans la douille (17) et entourant coaxialement l'extrémité distale de la tige de traction/poussée (4).

22. Instrument médical à corps allongé tubulaire selon la revendication 21, **caractérisé en ce que** la tige de traction/poussée (4) présente, dans la zone de son extrémité distale montée dans l'insert de guidage (31), une goupille de verrouillage (32) agencée transversalement à l'axe longitudinal de la tige de traction/poussée (4).

23. Instrument médical à corps allongé tubulaire selon la revendication 22, **caractérisé en ce que** la goupille de verrouillage (32) est montée librement mobile dans un alésage (33) de la tige de traction/poussée (4), de façon telle que les extrémités libres de la goupille de verrouillage (32) traversent des voies de guidage (34) réalisées dans l'insert de guidage (31).

24. Instrument médical à corps allongé tubulaire selon la revendication 22 ou la revendication 23, **caractérisé en ce que** les extrémités libres de la goupille de verrouillage (32) sont réalisées en tant que renflements (35) en forme de tête de boulon.
